# EUROPEAN PATENT APPLICATION

(11) **EP 4 552 584 A2**
(43) Date of publication of application: **14.05.2025**
(21) Application number: 25158376.1
(22) Date of filing: 16.11.2020
(51) Int. Cl.: A61B 17/00

(54) **LEFT ATRIAL APPENDAGE DEVICE**

(30) Priority: 15.11.2019 US 201962936045 P; 15.11.2019 US 201962936056 P; 25.06.2020 US 202063044234 P; 25.06.2020 US 202063044246 P
(62) Divisional of application: 20823998.8
(71) Applicant: W. L. Gore & Associates, Inc., Newark, DE 19711 (US)
(72) Inventor: FOX, Aaron D, Newark, DE 19711 (US); KOREY, Nathan C, Newark, DE 19711 (US); RUST, Keith O, Newark, DE 19711 (US); SILVERMAN, James D, Newark, DE 19711 (US); WHAM, Brett J, Newark, DE 19711 (US); WOLFE, Roark N, Newark, DE 19711 (US)
(74) Representative: HGF

(57) **Abstract**

Various aspects of the present disclosure are directed toward apparatuses, systems, and methods for placement in vessels, appendages, and openings in a body.

## Description

### CROSS-REFERENCE TO RELATED APPLICATION

This application claims the benefit of Provisional Application No. 62/936,045, filed November 15, 2019, Provisional Application No. 62/936,056, filed November 15, 2019, Provisional Application No. 63/044,234, filed June 25, 2020, and Provisional Application No. 63/044,246, filed June 25, 2020, all of which are incorporated herein by reference in their entireties for all purposes.

### FIELD

The present disclosure relates to implantable medical devices with movable fixation that may be used to occlude, filter and/or support apertures, conduits, spaces, organs, and other structures and/or openings within a patient.

### BACKGROUND

Cardiac structures such as atrial appendages can contribute to cardiac blood flow disturbance, which is associated with a number of cardiac-related pathologies. For example, complications caused by blood flow disturbance within an appendage and associated with atrial fibrillation can contribute to embolic stroke.

### SUMMARY

According to one example ("Example 1"), a device for placement in vessels, appendages, and openings in a body includes a frame having a plurality of elongate members, the frame including: a substantially uniform proximal end of the frame having (i) a center frame portion arranged at the proximal end and (ii) face portions of the plurality of elongate members extending from the center frame portion and including a curved pattern arranged substantially within a first plane approximately perpendicular to a longitudinal axis of the frame; a body portion; a membrane coupled to at least the substantially uniform proximal end of the frame; and a coating arranged on at least a portion of the membrane configured to minimize the thrombogenic response of blood contact with the membrane.

According to another example ("Example 2"), further to the device of Example 1, wherein the coating comprises heparin configured to lessen blood coagulation along the membrane coupled to at least the substantially uniform proximal end of the frame.

According to another example ("Example 3"), further to the device of Example 1, the coating is configured to decrease platelet adhesion to an exterior surface of the membrane coupled to at least the substantially uniform proximal end of the frame.

According to another example ("Example 4"), further to the device of Example 1, the coating is a carmeda heparin coating.

According to another example ("Example 5"), further to the device of Example 1, the plurality of elongate members are configured to angularly displace the center frame portion by between about 30 degrees and about 45 degrees in response to a compressive force applied to the body portion of the frame.

According to another example ("Example 6"), further to the device of Example 1, the plurality of elongate members are configured to angularly displace the center frame portion by between about 30 degrees and about 45 degrees in response to the compressive force reducing the circumference of the body portion of the frame by between about 20% and about 50%.

According to another example ("Example 7"), further to the device of Example 6, the plurality of elongate members are configured to angularly displace the center frame portion and maintain the center frame portion approximately aligned with a longitudinal axis of the frame in response to the compressive force.

According to another example ("Example 8"), further to the device of Example 1, the face portions of the plurality of elongate members include a first curved section, a second curved section, and an inflection point between the first curved section and the second curved section within the proximal end of the frame.

According to one example ("Example 9"), a method of deploying an implantable medical device within a left atrial appendage of a patient includes positioning a delivery sheath adjacent an ostium of the left atrial appendage; deploying an implantable medical device within the left atrial appendage from the delivery sheath, the implantable medical device having a frame having a plurality of elongate members, the frame including: a substantially uniform proximal end of the frame having (i) a center frame portion arranged at the proximal end and (ii) face portions of the plurality of elongate members extending from the center frame portion, and a body portion, and a membrane coupled to at least the substantially uniform proximal end of the frame, a coating arranged on at least a portion of the membrane configured to minimize the thrombogenic response of blood contact with the membrane; and lessening thrombosis blood coagulation along the membrane coupled to at least the substantially uniform proximal end of the frame.

According to another example ("Example 10"), further to the method of Example 9, the coating comprises heparin configured to lessen blood coagulation along the membrane coupled to at least the substantially uniform proximal end of the frame.

According to another example ("Example 11"), further to the method of Example 9, the coating is configured to decrease platelet adhesion to an exterior surface of the membrane coupled to at least the substantially uniform proximal end of the frame.

According to another example ("Example 12"), further to the method of Example 9, the method also includes absorbing one or more forces from the left atrial appendage with the plurality of elongate members by angularly displacing the center frame portion by between about 30 degrees and about 45 degrees in response to a compressive force applied to the body portion of the frame.

According to another example ("Example 13"), further to the method of Example 12, the method also includes angularly displacing the center frame portion in response to a compressive force applied to the body portion of the frame lessens longitudinal movement of the proximal end in response thereto.

According to another example ("Example 14"), further to the method of Example 13, the method also absorbing one or more forces from the left atrial appendage comprises mitigating thrombosis formation by maintaining a substantially uniform shape of the proximal end.

According to another example ("Example 15"), further to the method of Example 12, the plurality of elongate members are configured to angularly displace the center frame portion by between about 30 degrees and about 45 degrees in response to the compressive force reducing the circumference of the body portion of the frame by between about 20% and about 50%.

According to one example ("Example 16"), a device for placement in vessels, appendages, and openings in a body includes a frame having a plurality of elongate members, the frame including: a substantially uniform proximal end of the frame having (i) a center frame portion arranged at the proximal end and (ii) face portions of the plurality of elongate members extending from the center frame portion and including a curved pattern arranged substantially within a first plane approximately perpendicular to a longitudinal axis of the frame, a shoulder portion including first curved portions of the plurality of elongate members transitioning from the first plane to align approximately parallel with the longitudinal axis of the frame, and a first body portion including a first plurality of cells arranged circumferentially about the frame formed by first body portions of the plurality of elongate members, a hip portion including second curved portions of the plurality of elongate members transitioning the frame inwardly toward the longitudinal axis of the frame, a second body portion including a second plurality of cells arranged circumferentially about the frame; and a membrane attached to at least a portion of the frame and including a plurality of openings arranged circumferentially about the frame; and a plurality of anchors arranged at the hip portion configured to rotate relative to and toward the longitudinal axis and pass through the plurality of openings.

According to another example ("Example 17"), further to the device of Example 16, the plurality of anchors are configured to rotate and pass through the plurality of openings in response to constriction of the substantially uniform proximal end of the frame.

According to another example ("Example 18"), further to the device of Example 16, the plurality of anchors are configured to rotate pass through the plurality of openings in in response to the frame being arranged in a delivery configuration to avoid contact between an anchor tip and a delivery sheath.

According to one example ("Example 19"), a method of reducing thrombus arising from occluding a left atrial appendage of a patient including placing the implantable medical device across an ostium of the left atrial appendage, the implantable medical device having a frame with a substantially uniform proximal end and a membrane coupled to at least the substantially uniform proximal end of the frame and a coating arranged on at least a portion of the membrane configured to minimize the thrombogenic response of blood contact with the membrane.

According to another example ("Example 20"), further to the method of Example 19, the coating comprises heparin configured to lessen blood coagulation along the membrane coupled to at least the substantially uniform proximal end of the frame.

According to another example ("Example 21"), further to the method of Example 9, the coating is configured to decrease platelet adhesion to an exterior surface of the membrane coupled to at least the substantially uniform proximal end of the frame.

According to one example ("Example 22"), a device for placement in vessels, appendages, and openings in a body includes a frame having a plurality of elongate members, the frame including: a substantially uniform proximal end of the frame having (i) a center frame portion arranged at the proximal end and (ii) face portions of the plurality of elongate members extending from the center frame portion and including a curved pattern arranged substantially within a first plane approximately perpendicular to a longitudinal axis of the frame, a body portion including first body portions of the plurality of elongate members arranged substantially parallel to the longitudinal axis of the frame and second body portions of the plurality of elongate members tapering the frame inwardly relative to the longitudinal axis; and a membrane attached to at least a portion of the frame; and a coating arranged on at least a portion of the membrane configured to minimize the thrombogenic response of blood contact with the membrane.

According to another example ("Example 23"), further to the device of Example 22, the coating comprises heparin configured to lessen blood coagulation along the membrane coupled to at least the substantially uniform proximal end of the frame.

According to another example ("Example 24"), further to the device of Example 22, the coating is configured to decrease platelet adhesion to an exterior surface of the membrane coupled to at least the substantially uniform proximal end of the frame.

According to another example ("Example 25"), further to the device of Example 22, the coating is a carmeda heparin coating.

According to one example ("Example 26"), a device for placement in vessels, appendages, and openings in a body includes a frame having a plurality of elongate members, the frame including: a substantially uniform proximal end of the frame having (i) a center frame portion arranged at the proximal end and (ii) face portions of the plurality of elongate members extending from the center frame portion and including a curved pattern arranged substantially within a first plane approximately perpendicular to a longitudinal axis of the frame, a body portion including first body portions of the plurality of elongate members arranged substantially parallel to the longitudinal axis of the frame and second body portions of the plurality of elongate members tapering the frame inwardly relative to the longitudinal axis; and a membrane attached to at least a portion of the frame and including a plurality of openings arranged circumferentially about the frame; and a plurality of anchors arranged at the hip portion configured to rotate relative to and toward the longitudinal axis and pass through the plurality of openings.

According to another example ("Example 27"), further to the device of Example 26, the plurality of anchors are configured to rotate and pass through the plurality of openings in response to constriction of the substantially uniform proximal end of the frame.

According to another example ("Example 28"), further to the device of Example 26, the plurality of anchors are configured to rotate pass through the plurality of openings in in response to the frame being arranged in a delivery configuration to avoid contact between an anchor tip and a delivery sheath.

According to one example ("Example 29"), a device for placement in vessels, appendages, and openings in a body includes a frame having a plurality of elongate members, the frame including: a substantially uniform proximal end of the frame having (i) a center frame portion arranged at the proximal end and (ii) face portions of the plurality of elongate members extending from the center frame portion; a body portion; and a membrane attached to the frame and the plurality of elongate members are configured to angularly displace the center frame portion by between about 30 degrees and about 45 degrees in response to a compressive force applied to the body portion of the frame.

According to another example ("Example 30"), further to the device of Example 29, the plurality of elongate members are configured to angularly displace the center frame portion by between about 30 degrees and about 45 degrees in response to the compressive force reducing the circumference of the body portion of the frame by between about 20% and about 50%.

According to another example ("Example 31"), further to the device of Example 30, the plurality of elongate members are configured to angularly displace the center frame portion and maintain the center frame portion approximately aligned with a longitudinal axis of the frame in response to the compressive force.

According to another example ("Example 32"), further to the device of Example 29, the face portions of the plurality of elongate members include a first curved section, a second curved section, and an inflection point between the first curved section and the second curved section within the proximal end of the frame.

According to another example ("Example 33"), further to the device of Example 32, the face portions of the plurality of elongate members each include a starting point at the center frame portion and an end point a perimeter of the proximal end and the starting point and the ending port are radially offset relative to a tangent between the starting point and the ending point.

According to another example ("Example 34"), further to the device of Example 33, the starting point of the face portions of the elongate members and the end point of the face portions of the elongate members are offset by about 40 degrees and about 60 degrees.

According to another example ("Example 35"), further to the device of Example 33, a tangent perpendicular to the starting point at the center frame portion of each of the face portions of the elongate members intersects the perimeter of the proximal end between two of the face portions of the plurality of elongate members.

According to another example ("Example 36"), further to the device of Example 32, the first curved section includes a first radius of curvature and the second curved section includes a second radius of curvature and the first radius of curvature and the second radius of curvature are approximately equal.

According to another example ("Example 37"), further to the device of Example 32, the first curved section and the second curved section are in opposite directions.

According to another example ("Example 38"), further to the device of Example 29, the body portion includes a distal section including one or more circumferential members.

According to another example ("Example 39"), further to the device of Example 29, the circumferential members in the distal section facilitate uniform compression of the frame.

According to another example ("Example 40"), further to the device of Example 29, one or more of the plurality of elongate members include sections of lesser width within the body portion.

According to another example ("Example 41"), further to the device of Example 29, one or more of the plurality of elongate members include sections of lesser width within the proximal end.

According to another example ("Example 42"), a device for placement in vessels, appendages, and openings in a body includes a frame having a plurality of elongate members, the frame including: a substantially uniform proximal end of the frame having (i) a center frame portion arranged at the proximal end and (ii) face portions of the plurality of elongate members extending from the center frame portion and including a first curvature and a second curvature between the center frame portion and a perimeter of the proximal end with the first curvature being opposite the second curvature, and a body portion; and a membrane attached to the frame; wherein the face portions of the plurality of elongate members are configured to angularly displace the center frame portion and maintain the proximal end in a substantially uniform shape response to a compressive force applied to the body portion of the frame.

According to another example ("Example 43"), further to the device of Example 43, the plurality of elongate members are configured to angularly displace the center frame portion by between about 30 degrees and about 45 degrees in response to the compressive force reducing the circumference of the body portion of the frame by between about 20% and about 50%.

According to another example ("Example 44"), further to the device of Example 43, the plurality of elongate members are configured to angularly displace the center frame portion and maintain the center frame portion approximately aligned with a longitudinal axis of the frame in response to the compressive force.

According to one example ("Example 45"), a method of deploying an implantable medical device within a left atrial appendage of a patient includes positioning a delivery sheath adjacent an ostium of the left atrial appendage; deploying an implantable medical device within the left atrial appendage from the delivery sheath, the implantable medical device having a frame having a plurality of elongate members, the frame including: a substantially uniform proximal end of the frame having (i) a center frame portion arranged at the proximal end and (ii) face portions of the plurality of elongate members extending from the center frame portion, and a body portion, and a membrane attached to the self-expanding frame; and absorbing one or more forces from the left atrial appendage with the plurality of elongate members by angularly displacing the center frame portion by between about 30 degrees and about 45 degrees in response to a compressive force applied to the body portion of the frame.

According to another example ("Example 46"), further to the method of Example 45, angularly displacing the center frame portion in response to a compressive force applied to the body portion of the frame lessens longitudinal movement of the proximal end in response thereto.

According to another example ("Example 47"), further to the method of Example 46, absorbing one or more forces from the left atrial appendage comprises mitigating thrombosis formation by maintaining a substantially uniform shape of the proximal end.

According to another example ("Example 48"), further to the method of Example 45, the plurality of elongate members are configured to angularly displace the center frame portion by between about 30 degrees and about 45 degrees in response to the compressive force reducing the circumference of the body portion of the frame by between about 20% and about 50%.

The foregoing Examples are just that, and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1A is a cross-sectional view of a human heart in which a catheter delivery system is positioned in preparation for deployment of an implantable medical device into a left atrial appendage ("LAA") of the heart, in accordance with various aspects of the present disclosure.
FIG. 1B shows the configuration of FIG. 1A with the implantable medical device deployed from the catheter delivery system and positioned within the LAA, in accordance with various aspects of the disclosure.
FIG. 1C shows the configuration of FIG. 1A with the implantable medical device deployed from the delivery system and positioned within a vessel, in accordance with various aspects of the present disclosure.
FIG. 2 shows an example frame for an implantable medical device, in accordance with various aspects of the present disclosure.
FIG. 3 shows a perspective view of an example frame for an implantable medical device, in accordance with various aspects of the present disclosure.
FIG. 4 shows a top down view of a proximal end portion of an example frame for an implantable medical device, in accordance with various aspects of the present disclosure.
FIG. 5A shows a side view of example frame for an implantable medical device in response to a compressive force, in accordance with various aspects of the present disclosure.
FIG. 5B shows a top view of the example frame shown in FIG. 5A in a, in accordance with various aspects of the present disclosure.
FIG. 6 shows another example frame for an implantable medical device, in accordance with various aspects of the present disclosure.
FIG. 7 shows a close-up view of a portion of an example frame for an implantable medical device, in accordance with various aspects of the present disclosure.
FIG. 8 shows a close-up view of another portion of an example frame for an implantable medical device, in accordance with various aspects of the present disclosure.
FIG. 9 shows a close-up view of a cut pattern for an example frame for an implantable medical device, in accordance with various aspects of the present disclosure.
FIG. 10 shows an example delivery sheath, collet and a portion of an example frame for an implantable medical device, in accordance with various aspects of the present disclosure.
FIG. 11A is a first perspective view of an example implantable medical device, in accordance with various aspects of the present disclosure.
FIG. 11B is a side view of the implantable medical device, shown in FIG. 11A, in accordance with various aspects of the present disclosure.
FIG. 11C is a second perspective view of the implantable medical device, shown in FIGS. 11A-C, in accordance with various aspects of the present disclosure.
FIGS. 12A-D show example implantable medical devices of varying sizes, in accordance with various aspects of the disclosure.
FIGS. 13A-B show example implantable medical devices having a coating after implantation, in accordance with various aspects of the disclosure.
FIG. 14A shows an example frame and anchors with a delivery catheter in a first configuration, in accordance with various aspects of the disclosure.
FIG. 14B shows the frame, anchors, and the delivery catheter, as shown in FIG. 14A, in a second configuration, in accordance with various aspects of the disclosure.
FIG. 15A is an example illustration of a human heart in which an implantable medical device is arranged within a left atrial appendage ("LAA") of the heart, in accordance with various aspects of the present disclosure.
FIG. 15B shows a close-up illustration of the device and LAA, shown in FIG. 15A, in accordance with various aspects of the disclosure.
FIG. 16A is a representative SEM image (1000X) of the face of a device uncoated implant, in accordance with various aspects of the disclosure.
FIG. 16B is a representative SEM image (1000X) of the face of a device implant with the CBAS^{®} Heparin Surface, in accordance with various aspects of the disclosure.
FIG. 17 shows another example frame for an implantable medical device, in accordance with various aspects of the present disclosure.

### DETAILED DESCRIPTION

### Definitions and Terminology

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

### Description of Various Embodiments

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

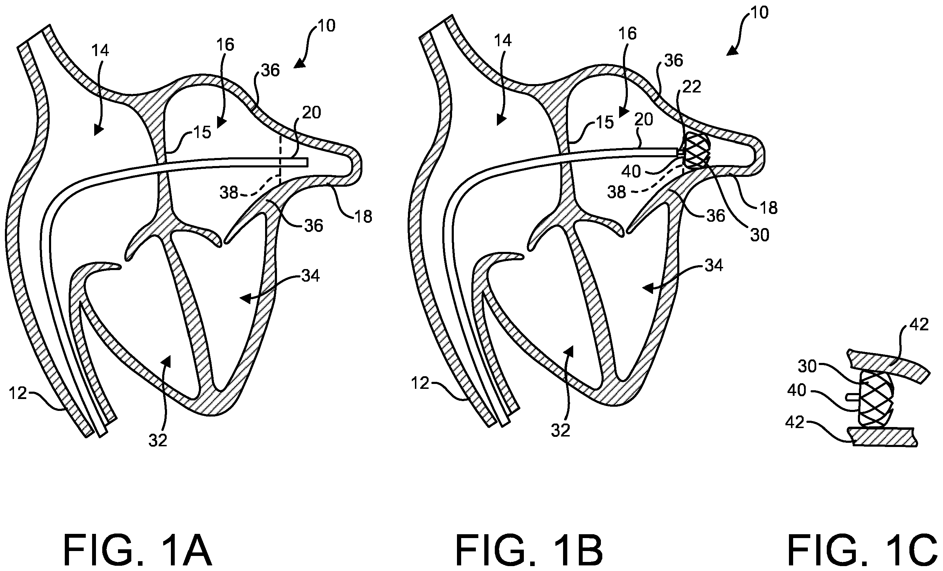

FIGs. 1A-B are a cross-sectional views of a human heart 10 in which a delivery sheath 20 is positioned in preparation for deployment of an implantable medical device 30 into an appendage 18 of the heart, in accordance with various aspects of the present disclosure. FIGs. 1A-B show a depiction of a right atrium 14, a left atrium 16, a right ventricle 32, and a left ventricle 34 of the heart 10. As is shown, the appendage 18 is located in the left atrium 16 of the heart 10, and thus, the appendage 18 may be considered the left atrial appendage 18. Although the following discussion focuses on deployment of the implantable medical device 30 into the left atrial appendage 18, the implantable medical device 30 may be deployed in other appendages or openings within the human heart 10 or in other locations of the human body.

The left atrial appendage 18 may be considered a muscular pouch extending from the anterolateral wall 36 of the left atrium 16 of the heart 10, which serves as a reservoir for the left atrium 16. In a normal cardiac cycle, the left atrial appendage 18 may contract rhythmically with the rest of the left atrium 16 during contraction of the heart 10. Thus, during a normal cardiac cycle, the left atrial appendage 18 contracts with the left atrium 16 and pumps blood that may gather or collect within the left atrial appendage 18 to circulate therefrom. However, during cardiac cycles characterized by arrhythmias (e.g., atrial fibrillation), the left atrial appendage 18 may fail to sufficiently contract along with the left atrium 16, which can allow blood to stagnate within the left atrial appendage 18. Stagnant blood within the atrial appendage 18 is susceptible to coagulating and forming a thrombus, which can dislodge from the atrial appendage 18 and ultimately result in an embolic stroke. The implantable medical device 30, consistent with various aspects of the present disclosure, may be delivered to the left atrial appendage 18 to help prevent and militate against blood stagnation within the left atrial appendage 18.

In certain instances and as is shown in FIGs. 1A-B, the implantable medical device 30 may be delivered to the left atrial appendage 18 by way of a minimally invasive transcatheter procedure. More specifically, the delivery sheath 20 may be navigated through a vena cava 12, into the right atrium 14, through an atrial septum 15, and into the left atrium 16 towards the appendage 18. In some implementations, the percutaneous access to the patient's vasculature can be at the patient's femoral vein, for example. It should be understood that this example technique is merely one example, and many other access techniques can also be performed to deploy the occlusive devices provided herein. At this point of the deployment process, the occlusive device is contained within a lumen of the delivery sheath 20, and is configured in a collapsed low-profile delivery configuration. Although transcatheter systems are generally shown and described, other delivery systems (e.g., thoracoscopic) are also contemplated.

FIG. 1B shows the configuration of FIG. 1A with the implantable medical device 30 deployed from the delivery sheath 20 and positioned within the left atrial appendage 18, in accordance with various aspects of the present disclosure. As shown, an inner catheter 22 may releasably couple to the implantable medical device 30, and is slidably disposed within the lumen of the delivery sheath 20. The inner catheter 22 can be used by a clinician operator to make the implantable medical device 30 deploy from the delivery sheath 20. For example, after positioning the implantable medical device 30 through an ostium 38 of the left atrial appendage 18, the clinician operator can retract the delivery sheath 20 in relation to the inner catheter 22 to unsheath and deploy the implantable medical device 30. The ostium 38 may be considered a portion of the anterolateral wall 36 of the left atrium 16 from which a taper originates to form the pouch-like structure of the left atrial appendage 18. The implantable medical device 30 may include an occlusive face 40 that is arranged near the ostium 38 of the left atrial appendage 18. The inner catheter 22 may releasably couple to the implantable medical device 30 via a hub or center frame portion arranged centrally within the occlusive face 40 of the implantable medical device 30.

After emerging from the constraining confines of the delivery sheath 20, the implantable medical device 30 can reconfigure to an expanded configuration. The implantable medical device 30 may expand to conform to the contours of the space defined within the left atrial appendage 18. The devices provided herein can be used in many different areas of the body, and that deployment of the implantable medical device 30 into the left atrial appendage 18 is merely one example implementation. More specifically, FIG. 1C shows the configuration of FIG. 1A with the implantable medical device 30 deployed from the delivery system and positioned within a vessel between the vessel walls 42, in accordance with various aspects of the present disclosure. At each implant location, forces (such as blood pumping or muscles contracting) acting on the implantable medical device 30 may threaten to dislodge the implantable medical device 30 from the implant location.

In certain instances, positioning of the implantable medical device 30 relative to the ostium 38 of the left atrial appendage 18 may be enhanced and ensures that the implantable medical device 30 prevents thrombus from embolizing from the left atrial appendage 18. More specifically, the occlusive face 40 may be arranged within the left atrial appendage 18 such that the occlusive face 40 connects portions of the anterolateral wall 36 on opposite sides of the ostium 38 to form a substantially uniform surface. In certain instances, blood may collect or stagnate along the face of a device implanted therein if the occlusive face 40 is non-uniform (e.g., a device having a hub that protrudes beyond other portions of the occlusive face; a device having an occlusive face that is concave, partially concave, or includes depressions, or a device having an occlusive face that is concave, partially concave) relative to the ostium 38 of the left atrial appendage 18 or the occlusive face includes protuberances. In these instances, thrombus may occur along the face of the implantable medical device 30 as a non-uniform surface may alter/disrupt the blood flow within the left atrium 18. Thus, a patient may remain susceptible to blood coagulation and thrombus formation if an implantable medical device 30 includes a non-uniform surface as the result of improper positioning or the design of the device.

After proper positioning and delivery of the implantable medical device 30, the inner catheter 22 can be decoupled from the implantable medical device 30, and the delivery sheath 20 and inner catheter 22 can be removed from the patient. With the implantable medical device 30 deployed as shown, the space defined within the left atrial appendage 18 is essentially separated from the left atrium 16 by virtue of the physical barrier provided by the implantable medical device 30. In this manner, stagnant blood within the LAA 18 that is susceptible to coagulating and forming thrombi may be prevented from entering the left atrium 16, and thereby prevented from potentially causing an embolic stroke. In addition, positioning of the occlusive face 40 of the implantable medical device 30 relative to the ostium 38 of the left atrial appendage 18 may help prevent blood collecting or stagnating along the face of the implantable medical device 30.

The implantable medical device 30 may include a frame element and a membrane coupled or attached to at least a portion of the frame. As discussed in further detail below, the membrane is configured to allow the inner catheter 22 to interface with the implantable medical device 30 during positioning. In addition, the membrane is also configured to maintain a uniform occlusive face 40. Thrombus may occur along the occlusive face 40 of the implantable medical device 30 as a non-uniform surface may alter/disrupt the blood flow within the left atrium 18. Thus, the implantable medical device 30, and system for deployment of the device 30, may be configured to maintain a uniform face 40 to lessen the opportunity for blood coagulation and thrombus formation.

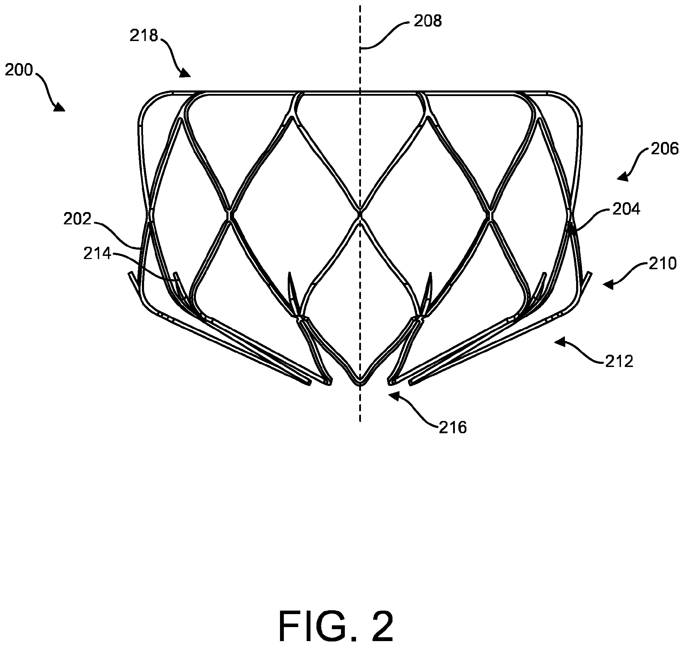

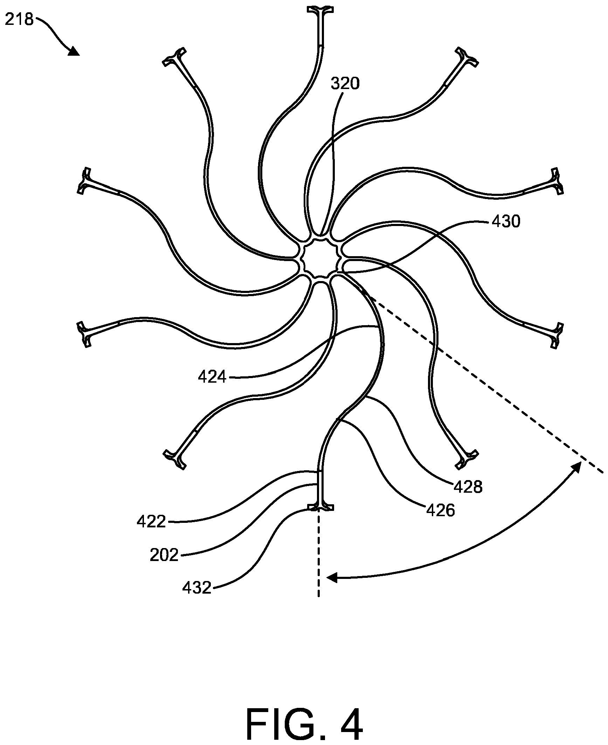

FIG. 2 shows an example frame 200 for an implantable medical device, in accordance with various aspects of the present disclosure. The implantable medical device may be a device for placement in vessels, appendages, and openings in a body. The frame 200 may be a unitary frame formed of a plurality of elongate members 202. In certain embodiments, the frame 200 may be unitary and self-expanding. The frame 200 may include body portions that have different shapes, angles, or other features (as explained in further detail with reference to FIG. 4) or may be another shape such as cylindrical, conical, frustoconical, hemispherical, a spherical cap, pyramidal, truncated pyramidal, and the like, and combinations thereof. Any and all combinations and sub-combinations of such varying shapes and varying geometries of shapes are envisioned and within the scope of this disclosure.

The frame 200 may include any number of rows and cells formed by the elongate members 202 within a body portion 206. The elongate members 202 within the body portion 206 may form multiple cells in a row. A single cell 204 is highlighted (as shown, the frame 200 includes multiple similar cells). The cell(s) 204 may be formed of a five-sided shape, a six-sided shape, or other shapes such as, but not limited to, polygonal, square, rectangular, parallelogram-shaped, rhomboidal, trapezoidal, diamond-shaped, chevron-shaped, octagonal, triangular, and the like. As shown in FIG. 2, the frame 200 tapers inwardly relative to a longitudinal axis 208 of the frame 200 from the body portion 206. The point at which the frame 200 transitions to the taper is a waist portion 210 of the frame 200 with an end portion 212 of the frame 200 being distal to the waist portion 210. The elongate members 202 also form the end portion 212. In certain instances, the elongate members 202 converge at the waist portion 210 of the frame 200 and diverge or bifurcate within the end portion 212 of the frame 200.

In addition to transitioning the frame 200 to a tapered portion, one or more anchors 214 may also be located at the waist portion 210. The one or more anchors 214 may be located at a portion of the cell 204 near or at which members 202 converge. The one or more anchors 214, arranged along the waist portion 210 configured to move inwardly (*e.g*., rotate) relative to and toward the longitudinal axis in response to the frame 200 being arranged in a delivery configuration. The one or more anchors 214 may retract, move or rotate inwardly such that when the frame 200 is loaded into a delivery catheter and into the delivery configuration (*e.g*., collapsed for transcatheter delivery). The frame 200 also includes a distal opening 216 at a distal end of the frame. The elongate members 202 taper inwardly within the end portion 212 of the frame and terminate or end at the distal opening 216. In certain instances, the distal opening 216 may include a diameter that is about between 10% and 20% of a diameter of the body portion 206. The distal opening 216 may be approximately about 5 mm and about 7 mm or more specifically about 6 mm in certain instances. Sizing of the distal opening 216 may facilitate uniform collapsing of a frame to a delivery configuration when arranged with a delivery sheath.

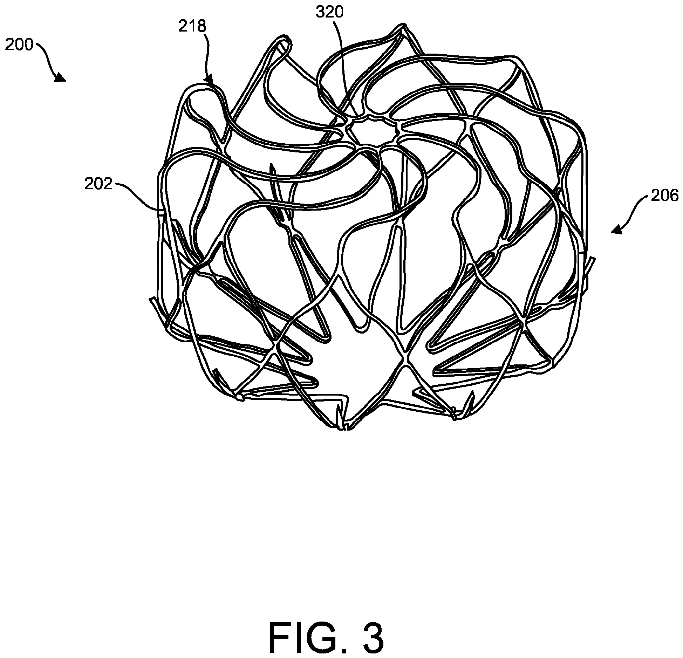

The frame 200 also includes a proximal end portion 218 formed by the elongate members 202 extending from a center frame portion (as shown in further detail in FIG. 3). In certain instances, the elongate members 202 bifurcate after transitioning from the proximal end portion 218 to the body portion 206 of the frame 200. In addition and as shown, the proximal end portion 218 of the frame 200 is uniform. In certain instances, the proximal end portion 218 of the frame 200 being uniform may include a substantially uniform surface. In addition, the proximal end portion 218 of the frame 200 being uniform may include instances where the elongate members 202 may form a unitary surface (*e.g*., within a common plane) across the proximal end portion 218 of the frame 200. In certain instances, the elongate members 202 are within the proximal end portion 218 of the frame 200 are arranged perpendicular to the waist portion 210. In certain instances, the proximal end portion 218 of the frame 200 may be without protrusions or without abrupt changes in shape. For example, the proximal end portion 218 of the frame 200 may include a gradual upward or downward slope increase in height relative to the longitudinal axis 208 of the frame 200 (*e.g*., about between 0.05 mm and about 2 mm increase or decrease) while maintaining a substantially uniform surface.

In addition, the elongate members 202 may transition from a plane perpendicular to the longitudinal axis 208 of the frame 200 into a plane that is parallel with the longitudinal axis 208 of the frame 200 in the body portion 206. When covered with a membrane (*e.g*., as shown in FIGs. 1A-C), the proximal end 218 of the frame 200 form an occlusive face. In addition, the proximal end 218 of the frame 200 may be configured to maintain a substantially uniform occlusive face in response to compressive forces acting on the body portion 206 and/or the end portion 212 of the frame 200. As described in further detail below, the elongate members 202 and the center frame portion (not shown).

FIG. 3 shows a perspective view of an example frame 200 for an implantable medical device, in accordance with various aspects of the present disclosure. The frame 200 may be used as a device for placement in vessels, appendages, and openings in a body. The frame 200 includes a substantially uniform proximal end 218 forming a face portion that includes a center frame portion 320 arranged at or within the proximal end 218 of the frame 200 and a plurality of elongate members 202 extending from the center frame portion 320.

The frame 200 may also include a body portion 206 that is also formed by the elongate members 202. At least a portion of the body portion 206 extends substantially perpendicular to the proximal end 218 of the frame 200. The portions of the elongate members 202 within the proximal end 218 and the center frame portion 320 form the face portion of the frame 200. When covered with a membrane (a shown in FIGS. 1A-C) the elongate members 202 within the proximal end 202 and the center frame portion 320 form an occlusive face of an implantable medical device.

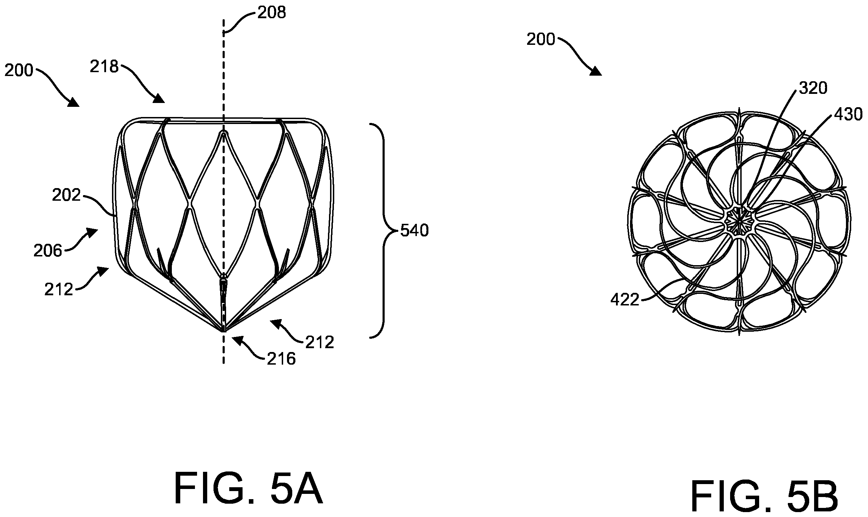

In certain instances, face portions of the plurality of elongate members 202 (within the proximal end portion 218) are configured to angularly displace the center frame portion 320 in response to a compressive force applied to the body portion 206 of the frame 200 as is explained in greater detail with reference to FIGS. 5A-B. The face portions of the plurality of elongate members 202 may be configured to angularly displace the center frame portion 320 to maintain the proximal end 218 of the frame 200 within a common plane. In certain instances, the proximal end 320 is substantially uniform in the absence of forces acting on the body portion 206 and/or edges or a perimeter of the proximal end 218 of the frame 200 and when forces are acting on the body portion 206 and/or edges or a perimeter of the proximal end 218 of the frame 200.

The face portions of the plurality of elongate members 202 are configured to absorb one or more forces from a location of implantation such as the left atrial appendage. The face portions of the plurality of elongate members 202 angularly displace the center frame portion in response the forces acting on the body portion of the frame 200. Absorbing one or more forces from the left atrial appendage includes mitigating thrombosis formation by maintaining a substantially uniform occlusive face. Maintaining the proximal end 218 as substantially uniform by way of the angular displacement of the center frame portion 320 lessens the opportunity for blood coagulation and thrombus formation. In certain instances, substantially uniform can include a surface that is without abrupt or random deviations along the surface of the proximal end 218.

FIG. 4 shows a top down view of a proximal end portion 218 of an example frame 200 for an implantable medical device, in accordance with various aspects of the present disclosure. Face portions 422 of the elongate members 202, as noted above, may be configured to maintain a substantially uniform shape of the proximal end 218 of the frame 200. For ease of illustration, one of the face portions 422 of the elongate members 202 are shown highlighted in FIG. 4.

Each of the face portions 422 of the elongate members 202 may include a first curved section 424, a second curved section 426, and an inflection point 428 between the first curved section 424 and the second curved section 426 within the proximal end 218 of the frame 200. In certain instances, the face portions 422 of the elongate members 202 include a starting point 430 at the center frame portion 320 and an end point 432 at a perimeter of the proximal end 218. The starting point 430 and the ending port 432 may be radially offset relative to a tangent between the starting point 430 and the ending port 430. In certain instances, the tangent, as shown, is perpendicular to the starting point 430 at the center frame portion 320 and the tangent intersects the perimeter of the proximal end 218 between adjacent ones of the face portions 422 of the elongate members 202.

In certain instances, the first curved section 424 includes a first radius of curvature and the second curved section 426 includes a second radius of curvature. The first radius of curvature and the second radius curvature of may be approximately equal. In addition and as shown, the first curved section 424 and the second curved section 426 may be in opposite directions. In addition, the radiuses of curvature may distribute strain across the face portions 422 of the elongate members 202. The strains that occur on the proximal end 218 may distribute evenly across the face portions 422 of the elongate members 202 in certain instances.

In certain instances, the starting point 430 of the face portions 422 of the elongate members 202 and the ending port432 of the face portions 422 of the elongate members 202 are offset by between about 40 degrees and about 60 degrees. As shown, the starting point 430 of the face portions 422 of the elongate members 202 and the ending port 432 of the face portions 422 of the elongate members 202 are offset by about 45 degrees (with about including +/- 5%). In addition, the starting point 430 of the face portions 422 of the elongate members 202 and the ending port 432 of the face portions 422 of the elongate members 202 are offset by about 55 degrees (with about including +/- 5%).

FIGS. 5A-B shows side and top views, respectively, of example frame 200 for an implantable medical device in response to a compressive force, in accordance with various aspects of the present disclosure. The implantable medical device may be a device for placement in vessels, appendages, and openings in a body. The frame 200 may be a unitary frame formed of a plurality of elongate members 202. In certain embodiments, the frame 200 may be unitary and self-expanding. As discussed in detail above, the frame 200 includes a body portion 206 and tapers inwardly relative to a longitudinal axis 208 of the frame 200 from the body portion 206. The point at which the frame 200 transitions to the taper is a waist portion 210 of the frame 200 with an end portion 212 of the frame 200 being distal to the waist portion 210. The frame 200 also includes a distal opening 216 at a distal end of the frame. The elongate members 202 taper inwardly within the end portion 212 of the frame and terminate or end at the distal opening 216. The frame 200 also includes a proximal end portion 218 formed by the elongate members 202 extending from a center frame portion 320 (shown in FIG. 5B).

Forces may act on the frame 200 when implanted in a patient. The frame 200 may alter in shape or configuration in response to compressive forces acting on the frame 200, an example of which is shown in FIGS. 5A-B. In certain instances and as shown, the frame 200, the plurality of elongate members 202 are configured to angularly displace the center frame portion 320 in response to a compressive force applied to the body portion 206 of the frame 200. By angularly displacing the center frame portion 320, face portions 422 of the elongate members 202 may be configured to maintain a substantially uniform shape of the proximal end 218 of the frame 200. For ease of illustration, one of the face portions 422 of the elongate members 202 are shown highlighted in FIG. 5B.

In certain instances and as shown in FIG. 5A, the proximal end portion 218 of the frame 200 maintains a uniform surface may include instances across the proximal end portion 218 of the frame 200 in response to the compressive forces as a result of the angular displacement by the elongate members 202. In certain instances, the elongate members 202 may angularly displace the center frame portion 320 by between about 30 degrees and about 45 degrees. The amount of angular displacement may be determined by viewing a starting point 430 at the center frame portion 320 of the face portions 422 of the elongate members 202. The starting point 430 (e.g., a peak or outwardly extending undulation of the center frame portion 320) may rotate about the longitudinal axis 208 in response to the compressive forces. In certain instances, the proximal end portion 218 of the frame 200 maintains a uniform surface with an outward or inward movement, relatively to the longitudinal axis 208, of less than 0.50 mm - 1.5 mm. For example, in some instances, upon a 25% radial compression of implantable device body, the proximal end portion 218 maintains uniformity without extending inward or outward, greater than about 0.75 mm. The inward our outward movement of the proximal end portion 218 may result in a curvature from perimeter (e.g., end point 432 shown in FIG. 4) of the proximal end portion 218. In these instances, the curvature of the proximal end portion 218 of the frame 200 is constant and without additional protrusions such that the proximal end portion 218 of the frame 200 maintains a uniform surface. Absorbing one or more forces from the left atrial appendage includes mitigating thrombosis formation by maintaining a substantially uniform occlusive face

In certain instances, plurality of elongate members 202 (e.g., face portions 422 of the elongate members 202) are configured to angularly displace the center frame portion 320 by between about 30 degrees and about 45 degrees in response to the compressive force reducing the circumference of the body portion 206 of the frame 200 by between about 20% and about 50%. For example, in some instances, the center frame portion 320 has an angular displacement of between about 33 degrees and about 35 degrees in response to a compressive force reducing the circumference by about 25%. In addition, the plurality of elongate members 202 may be configured to angularly displace the center frame portion 320 and maintain the center frame portion approximately aligned with a longitudinal axis 208 of the frame 202 in response to the compressive force. Further, the face portions 422 of the elongate members 202 may be configured to maintain spacing at the center frame portion 320 in response to the compressive force as shown in FIG. 5B.

In addition, the frame 200 may be configured to substantially maintain a length 540 of the frame 200 in response to compressive forces. As the face portions 422 of the elongate members 202 may be configured to absorb forces acting on the body to maintain the uniformity of the proximal end 218, the body portion 206, in certain instances, maintains the length 540 of the frame 200 within a percentage of the original length 540. In certain instances, the length 540 of the frame 200 is maintained between about 100%, about 105%, about 110%, about 115%, about 120%, about 125%, about 130%, or about 135% of the original length 540 in response to compressive forces.

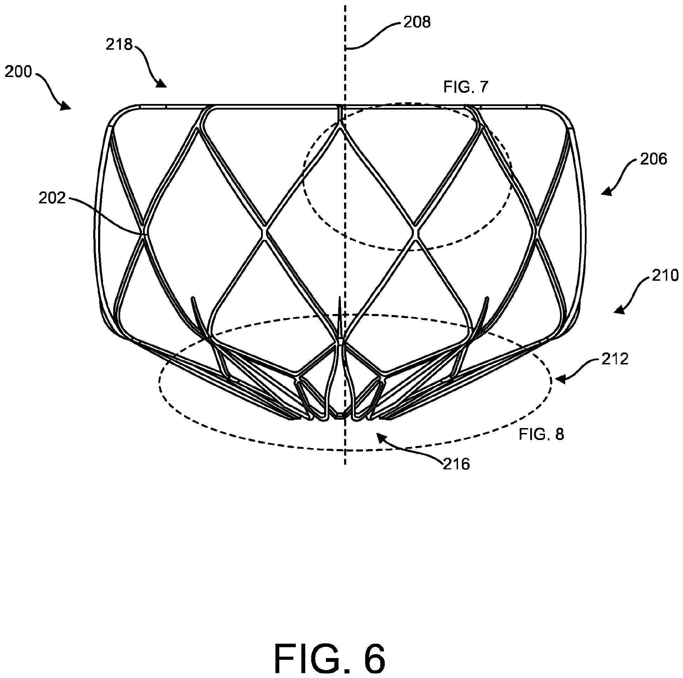

FIG. 6 shows another example frame 200 for an implantable medical device, in accordance with various aspects of the present disclosure. The implantable medical device may be a device for placement in vessels, appendages, and openings in a body. The frame 200 may be a unitary frame formed of a plurality of elongate members 202. The frame 200 may include body portions (e.g., body portion 206 and end portion 212) that have different shapes, angles, or other features or may be another shape such as cylindrical, conical, frustoconical, hemispherical, a spherical cap, pyramidal, truncated pyramidal, and the like, and combinations thereof. Any and all combinations and sub-combinations of such varying shapes and varying geometries of shapes are envisioned and within the scope of this disclosure.

The frame 200 also includes a proximal end portion 218 formed by the elongate members 202 extending from a center frame portion as explained in detail above. In certain instances, the elongate members 202 bifurcate after transitioning from the proximal end 218 to the body portion 206 of the frame 200. In addition and as shown, the proximal end portion 218 of the frame 200 is substantially uniform. The elongate members 202 may form a unitary surface (e.g., within a common plane) across the proximal end portion 218 of the frame 200. In certain instances, the elongate members 202 are within the proximal end portion 218 of the frame 200 are arranged perpendicular to the longitudinal axis 208. In addition, the elongate members 202 may transition from a plane perpendicular to the longitudinal axis 208 of the frame 200 into a plane that is parallel with the longitudinal axis 208 of the frame 200 in the body portion 206.

The frame 200 tapers inwardly relative to a longitudinal axis 208 of the frame 200 from the body portion 206. The point at which the frame 200 transitions to the taper is a waist portion 210 of the frame 200 with an end portion 212 of the frame 200 being distal to the waist portion 210. The elongate members 202 also form the end portion 212. In certain instances, the elongate members 202 converge at the waist portion 210 of the frame 200 and diverge or bifurcate within the end portion 212 of the frame 200.

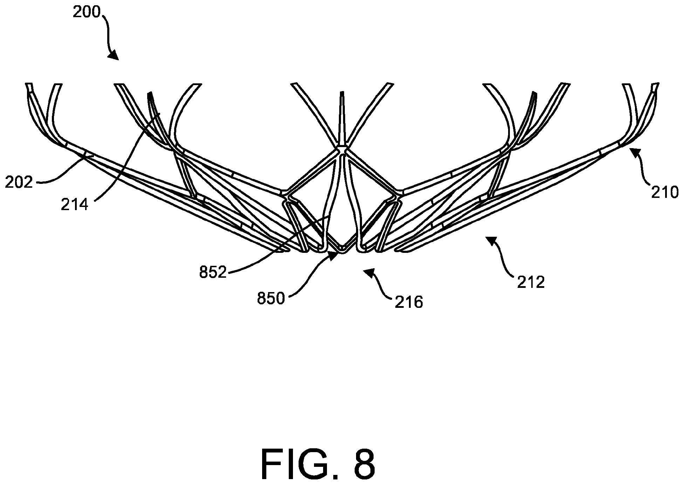

In addition, the elongate members 202 may include portions of greater width and less width than other portions of the elongate members 202. The elongate members 202 may include varying width depending on the location within the frame 200. For example, the elongate members 202 within the proximal end 218, the body portion 206, and/or the end portion 212 may have areas of differing widths as discussed in further detail and highlighted in FIG. 8. The end portion 212 may be shorter in length relative to the body portion 206.

The frame 200 also includes a distal opening 216 at a distal end of the frame. The elongate members 202 taper inwardly within the end portion 212 of the frame and terminate or end at the distal opening 216. The distal opening 216 may be sized, as explained in further detail below, relative to an outer diameter of the body portion 206 to facilitate deployment of the frame 200 as discussed in further detail and highlighted in FIG. 8.

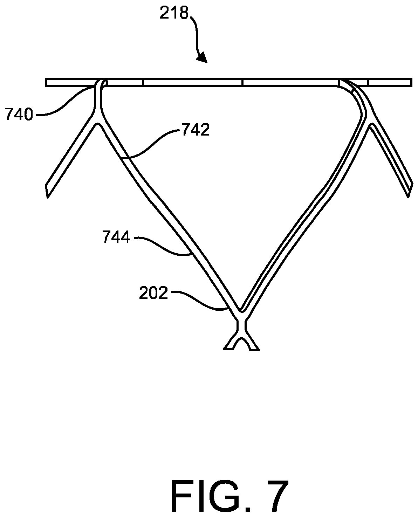

FIG. 7 shows a close-up view of a portion of an example frame 200 for an implantable medical device, in accordance with various aspects of the present disclosure. The portion of the body portion 206 of the frame 200 shown in FIG. 7 is immediately adjacent or below the proximal end 218 of the frame 200. As noted above with reference to FIG. 2, the elongate members 202 within the proximal end 218 may transition from a plane perpendicular to the longitudinal axis of the frame 200 to a plane that is parallel with the longitudinal axis 208 of the frame 200 in the body portion 206. At the perimeter of the proximal end 217 is a shoulder 740 of the device that is curved to transition the elongate members 202. For ease of illustration, the shoulder 740 of the frame 200 is highlighted once on FIG. 7, however, each elongate member 202 may include the shoulder 740 to transition the proximal end 218 to the body portion 206.

In certain instances, the elongate members 202 bifurcate within the body portion 206 immediately adjacent to or distal of the shoulder 740. The sections of the elongate members 202 immediately adjacent to or distal of the shoulder 740 may include sections of lesser width within the body portion 206. Other sections of the elongate members 202 may also include or alternatively include sections of lesser width.

As shown in FIG. 7, a first section 742 of the elongate members 202 has a less width than a second section 744 of the elongate members 202. The differing widths in this area of the body portion 206 may facilitate fatigue resistance of the frame 200. Forces acting on the body portion 206 may collect or be distributed toward the shoulder 740 of the frame 200. The differing widths in this area of the body portion 206 may facilitate fatigue resistance through absorption and distribution of forces through the elongate members 202.

FIG. 8 shows a close-up view of another portion of an example frame 200 for an implantable medical device, in accordance with various aspects of the present disclosure. Similar to the body portion 206, the end portion 212 of the frame 202 includes cells 850.

As shown, the frame 200 tapers inwardly toward an end portion 212 starting at the waist portion 210 with the elongate members 202 originating at adjacent anchors 214 converging at the distal opening 216. The frame 200 shown in FIG. 8 includes circumferential members 852 between the elongate members 202 originating at adjacent anchors 214 converge at the distal opening 216 within the cells 850. In certain instances, the circumferential members 852 facilitate uniform compression of the frame 200. In certain instances, the frame 200 may pleat when compressed in the absence of the circumferential members 852. The circumferential members 852 lessen the chance that the frame 200 pleats or non-uniformly reduces in diameter during device deployment, device loading, and device re-loading.

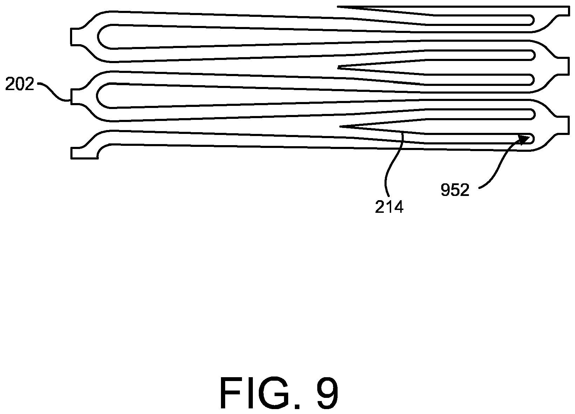

FIG. 9 shows a close-up view of a cut pattern for an example frame 200 for an implantable medical device, in accordance with various aspects of the present disclosure. The frame 200 adjacent the anchors 214 includes a radius 952 that separates the anchors 214 from the elongate members 202 adjacent either side of the anchors 214. The radius 952 may be semi-circular and configured to distribute strains or forces acting on the frame 200 adjacent the anchors 214.

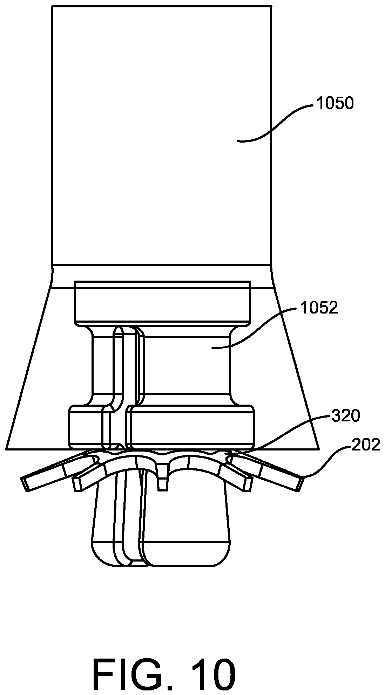

FIG. 10 shows an example delivery sheath 1050, collet 1052 and a portion of an example frame 200 for an implantable medical device, in accordance with various aspects of the present disclosure. The portion of the frame 200 shown in FIG. 10 is a center frame portion 320 and portions of elongate members 202 described in detail above.

The collet 1052 may form a portion of a delivery catheter and may interface with the center frame portion 320. The collet 1052 may be integrally formed or attached to a distal end of the delivery catheter (as shown in FIG. 1) arranged through the delivery sheath 1050. The collet 1052 may be configured to pass at least partially through and engage the center frame portion 320 to enable movement of the frame 200. The collet 1052 may be frictionally engaged with the center frame portion 320 and release in response to rotational movement of the delivery catheter. Movement of the collet 1052 moves the frame 200 into and out of the delivery sheath 1050. The collet 1052 may be configured to releasable couple to the frame 200 without use of a screw or other mechanical engagement to facilitate maintaining a substantially uniform proximal end portion as described in detail above.

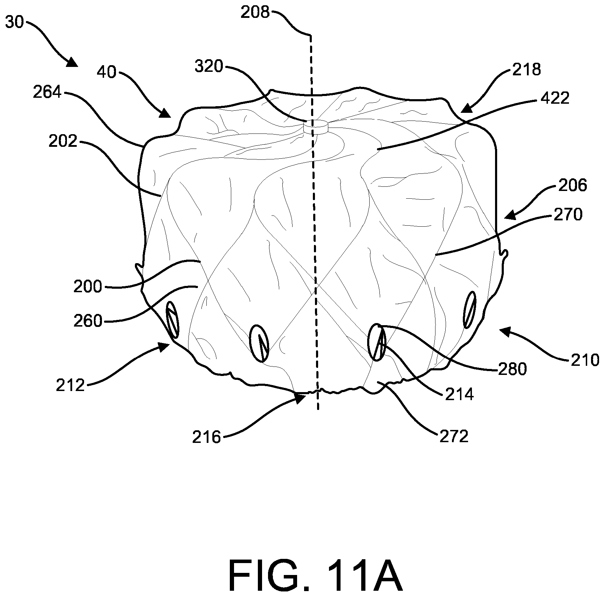

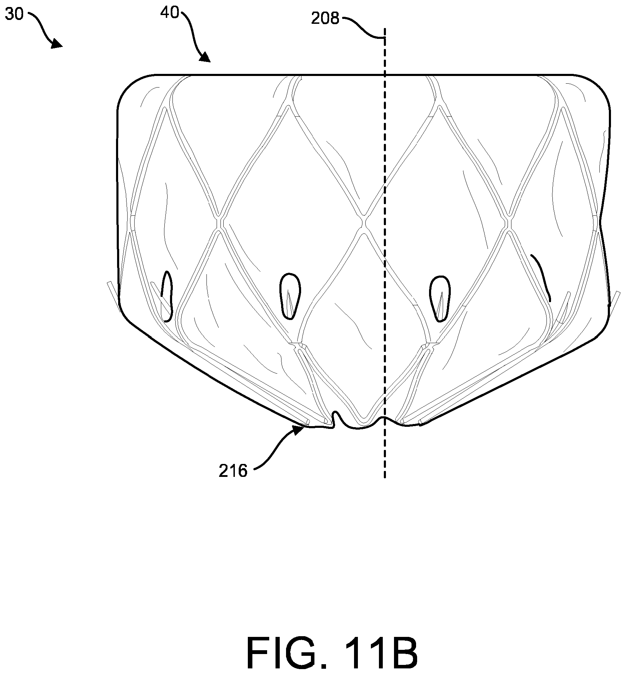

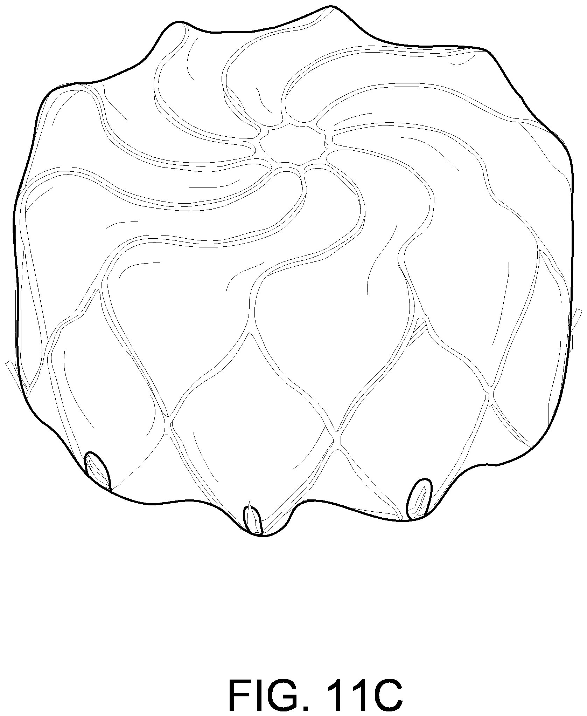

FIG. 11A is a first perspective view of an example implantable medical device 30, FIG. 11B is a side view of the implantable medical device 30 and FIG. 11C is a second perspective view of the implantable medical device 30, in accordance with various aspects of the present disclosure. The implantable medical device 30 includes a membrane 260 and a frame 200 (partially obscured by the membrane 260). The frame 200 may be formed by a plurality of elongate members 202. A proximal end 218 of the frame 200 includes a center frame portion 320. In addition, the proximal end 218 of the frame 200 may be hubless. The center frame portion 320 may may be configured to releasable couple to a delivery system (*e.g*., a collet 1052) without use of a screw or other mechanical engagement to facilitate maintaining a substantially uniform proximal end portion.

The proximal end 218 of the frame 200 may lack a feature that extends outwardly or inwardly relative to the proximal end 218 of the frame 200. Face portions 422 of the plurality of elongate members 202 may extend from the enter frame portion 208 as a substantially uniform surface (*e.g*., within a common plane) and form the occlusive face 40 along with the frame 200. As shown above, for example, with reference to FIG. 3, the face portions 422 of the plurality of elongate members 202 may include a curved pattern within a plane that includes the proximal end 218 of the frame 200. In certain instances, the proximal end 218 of the frame 200 may be within a common plane or include approximately between a 1% and 5% deviation inwardly or outwardly from the plane. In certain instances, the frame 200 may be formed of a metal or metallic material such as nitinol (NiTi). In certain more specific embodiments, the frame 200 may be formed from a single unitary piece of nitinol. The curved pattern of the face portions 422 of the plurality of elongate members 202 may facilitate conformability of the proximal end 218 of the frame (*e.g*., occlusive face 40 of the device 30). The face portions 422 of the plurality of elongate members 202 may absorb forces when the device is deployed within a patient such that the proximal end 218 is compliant to forces (*e.g*., soft) and conformable (*e.g*., responds and adapts to forces within the patient).

The implantable medical device 30, as shown in FIG, 11, may also include a first body portion 206 extending substantially parallel to the longitudinal axis 208 of the implantable medical device 30. A shoulder portion 262 of the frame 200 may transition the elongate members 202 from the plane of the proximal end 218 of the frame 200 to be aligned substantially parallel (*e.g*., within approximately between a 1% and 5% deviation from) the longitudinal axis 208 of the implantable medical device 30 (or frame 200). First body portions 270 of the elongate members 202 form the body portion 206.

The implantable medical device 30 may also include a waist portion 210 angled relative to the longitudinal axis 208. The waist portion 210 may transition the frame 200 to a second body portion 212 (or end portion of the frame 200 as discussed in detail above).The second body portion 212 converges the frame 200 inwardly toward the longitudinal axis 208. Each of the first body portion 206 and the second body portion 212 include a plurality of cells extending above the circumference of the frame 200 as shown and discussed above relative to in FIGS. 2-8, for example. Second body portions 272 of the elongate members 202 form the second body portion 212.

The frame 200 also includes a distal opening 216 at a distal end of the frame. The elongate members 202 taper inwardly within the end portion 212 of the frame and terminate or end at the distal opening 216. In certain instances, the distal opening 216 may include a diameter that is about between 10% and 20% of a diameter of the body portion 206.

As noted above, the implantable medical device 30 may be a device for placement in vessels, appendages, and openings in a body. The implantable medical device 30 may have a shape that mirrors or conforms to a target location into which it is implanted. For example and as shown in FIG. 2, the implantable medical device 30 includes an acorn-like shape when the implantable medical device 30 is configured for implantation into a patient's left atrial appendage. The implantable medical device 30 may have a tubular shape, a circular shape, or other shapes that are consistent with the target location into which the implantable medical device 30 is implanted.

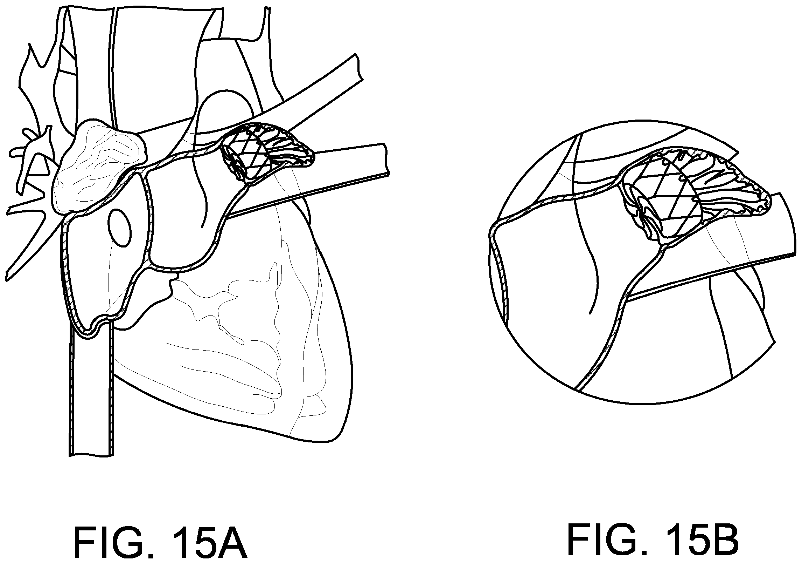

In certain instances, the membrane 260, may be coupled or attached to at least a portion of the frame 200. In certain instances, the membrane 260 may be coupled to only or at least and the proximal end 218 of the frame 200. The membrane 260 and the face portion 262 of the frame 200 form a substantially uniform occlusive face 40 with the membrane 260 extending across and covering the center frame portion 320. The proximal end 218 of the frame 200 may be disposed across an ostium of a target portion of a patient (such as the left atrial appendage as shown in FIG. 15A and FIG. 15B) and rapidly occlude and block the target portion behind the membrane 260. In addition, the membrane 260 may substantially or fully cover the proximal end 218 of the frame 200 such that there is no or minimal exposure to the frame 200. In certain instances and as discussed in further detail below, at least the membrane 260 that is in contact or coupled to the proximal end 218 of the frame 200 may include a coating that is configured to minimize a thrombogenic response that may occur due to exposure of the membrane 260 to blood contact.

The face portions 422 of the elongate members 202, as discussed in detail above, may be configured to deflect and maintain a substantially uniform shape or surface of the proximal end 218 of the frame 200. For ease of illustration, one of the face portions 422 of the elongate members 202 are shown highlighted in FIG. 4. The face portions 422 of the plurality of elongate members 202 are configured to absorb one or more forces from a location of implantation such as the left atrial appendage. The face portions 422 of the plurality of elongate members 202 angularly displace the center frame portion 320 in response the forces acting on the body portions 206, 212 of the frame 200. Absorbing one or more forces from the left atrial appendage may include mitigating thrombosis formation by maintaining a substantially uniform occlusive face and conforming the device to the left atrial appendage.

The membrane 260 may be attached to at least portions of the face portions 422 of the elongate members 202. In certain instances, the membrane 260 may be attached to at least a portion of each of the face portions 422 of the elongate members 202. The membrane 260 may be configured to deflect and maintain a substantially uniform surface and move along with the face portions 422 of the elongate members 202 in response to compressive forces acting on the body portion 212 of the frame 200. The membrane 260 may be configured to maintain a surface smoothness and move along with the face portions 422 of the elongate members 202 in response to compressive forces acting on the body portion 212 of the frame 200. The membrane 260, in certain instances, lacks surface roughness or substantial changes in surface topography to maintain the surface smoothness of the occlusive face 40. The membrane 260 being attached to at least a portion of each of the face portions 422 of the elongate members 212 may be configured to lessen billowing of the membrane 260 relative to the elongate members 212 and occlusive face 40.

The center frame portion 320 of the frame is configured to interface with a delivery system as described in further detail above with reference to FIG. 10 delivery of the implantable medical device 30 to the target location. To avoid thrombus formation, it may be beneficial for the implantable medical device 30 maintain the uniform occlusive face after delivery. In certain instances, the membrane 260 is configured to stretch within the frame 200 in response to interaction with a delivery system. In certain instances, the membrane 260 may be configured to recover from being deflected into the center frame portion 320 to maintain a substantially uniform occlusive face 40 with the proximal end 218 of the frame 200. After the delivery system has been removed from the center frame opening 208, the membrane 260 may be configured to rebound or move back in line with the proximal end 218 of the frame 200 (either an interior surface or exterior surface of the proximal end 218 of the frame 200) to deflect and maintain the substantially uniform occlusive face 40.

The membrane 260 may include a first layer and a second layer. In certain instances, the second layer may be attached to a percentage of the interior surface of the frame 200 that is less than an entirety of the interior surface. In certain instances, the second layer may be centered relative to the center frame portion 320. The second layer may be between about 25% and about 75% of the interior surface of the proximal end 218 of the frame 200. In certain instances, one or both of the first layer and the second layer of the membrane 260 is configured to reinforce and add elastomeric properties to the membrane 260. The elastomeric properties may be configured to facilitate the ability of the membrane 260 to stretch and rebound.

In addition to transitioning the frame 200 to a tapered portion, one or more anchors 214 may also be located at the waist portion 210. The one or more anchors 214 may be located at a portion of the cell near or at which members 202 converge. The one or more anchors 214, arranged along the waist portion 210 configured to move inwardly (*e.g*., rotate) relative to and toward the longitudinal axis in response to the frame 200 being arranged in a delivery configuration. The one or more anchors 214 may retract, move or rotate inwardly such that when the frame 200 is loaded into a delivery catheter and into the delivery configuration (*e.g*., collapsed for transcatheter delivery). In certain instances, the anchors 214 may retract, move or rotate inwardly such that when the occlusive face 40 is constricted.

In certain instances, the membrane 260 includes a plurality of openings 280 spaced above a circumference of the device 30. The one or more anchors 214 may be configured to move inwardly (*e.g*., rotate) relative through the one or more openings 280 in the membrane 260. The openings 280 in the membrane 260 may include a height that is approximately equal to or slightly larger than (*e.g*., between about 1% and about 5%) a height of the openings 280 in the membrane 260. The openings 280 in the membrane 260 may enable retraction or rotation of the anchors 214 without catching on, encumbering, or damaging the membrane 260.

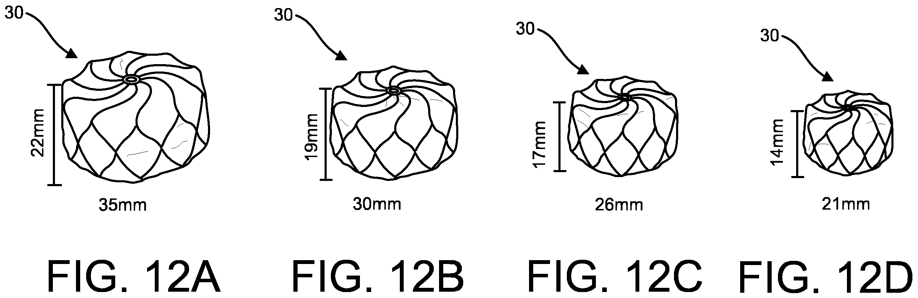

FIGS. 12A-D show example implantable medical devices 30 of varying sizes, in accordance with various aspects of the disclosure. As discussed above, the devices 30 may include ratios between a length of the device and an overall diameter of the devices 30 and/or a size of the distal opening. The four devices 30 shown may be configured for versatile sizing due to the conformability and adaptability of the devices 30 to a patient's left atrial appendage. For example, other devices may have up to eight difference diameter devices that are configured to fit the size ranges that the four devices 30 are intended for.

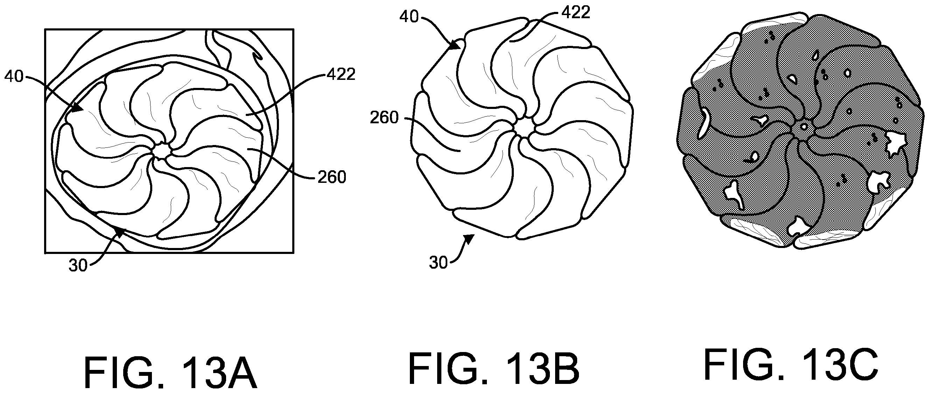

FIGS. 13A-B show example implantable medical devices 30 having a coating after implantation, in accordance with various aspects of the disclosure. FIG. 13C shows an example medical device 30 without a coating. As shown in FIGS. 13A-B, a membrane 260 is arranged or attached to a face portion 262 of face portions of elongate members 202 forming a substantially uniform occlusive face 40. As shown in FIG. 13A, the occlusive face 40 of the device 30 may be disposed across an ostium of a target portion of a patient (such as the left atrial appendage) and rapidly occlude and block the target portion behind the membrane 260. In addition and as is shown, the membrane 260 substantially or fully covers the occlusive face 40 that there is no or minimal exposure to the elongate members 202.

The membrane 260 of the devices 30 shown in FIGS. 13A-B include a coating that is configured to minimize a thrombogenic response that may occur due to exposure of the membrane 260 to blood contact. In certain instances, the coating may be a heparin coating. The heparin coating is utilized to bind heparin molecules to the membrane 260. For further reference regarding a heparin coated membrane 260, reference may be made to U.S. Patent No. 6,461,665 ("Scholander") for the specific teachings of antithrombogenic activity of surface immobilized heparin, which is incorporated herein by reference. In certain instances, the heparin coating may be CARMEDA^{®} BioActive Surface (CBAS^{®} Heparin Surface). As an example, the device 30 shown in FIG. 13A includes a CBAS^{®} Heparin Surface having been implanted in a canine for 180 days (and includes minimal/no thrombus on the surface of the membrane 260). The device 30 shown in FIG. 13B shows a device 30 that includes a CBAS^{®} Heparin Surface having been tested in the blood loop described below in the Test Methods section (and includes minimal/no thrombus on the surface of the membrane 260). FIG. 13C shows a device without an anti-thrombogenic coating such as heparin having been tested in the blood loop described below in the Test Methods section and shows substantial thrombus coverage.

In addition, the membrane 260 may be configured to inhibit, filter, modulate, or substantially modulate the passage of fluids and/or materials (such as blood and/or thrombus) through the membrane 260. In some embodiments, the membrane is configured to induce rapid tissue ingrowth therein. In certain instances, the membrane 260 provides for a blood or body fluid impermeable membrane that occludes the flow of blood or bodily fluids through the membrane 260 yet promotes the ingrowth and endothelialization. The membrane 260 can have a microporous structure that provides a tissue ingrowth scaffold for durable occlusion and supplemental anchoring strength of frames. In some embodiments, the membrane 260 may be a porous member. Pores of the membrane 260 may be sized to substantially, or in some examples completely, help prevent passage of blood, other bodily fluids, and emboli. In some implementations, the membrane prevents or substantially prevents passage of blood, other bodily fluids, thrombi, emboli, or other bodily materials through the membrane. In addition, the pores of the membrane 260 may facilitate attached or impregnation of heparin to lessen the opportunity for thrombus formation. Example testing parameters are set forth below that describe the methods and equipment used to show the thrombogenic responses of coated and uncoated membranes 260.

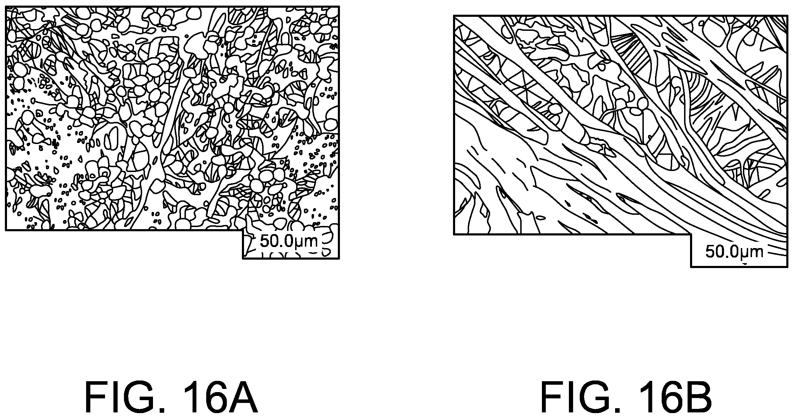

In certain instances, the heparin coating may be applied to the membrane 260 in one or more layers. The chemical constituents of the covering material in each layer can be the same or different. In some instances, the covering material is cross-linked to itself or other covering materials in other layers. The cross-linking bonds can be covalent or ionic. The heparin covering may form at least one layer on at least a portion of the membrane 260 and may cross-link to itself or other layers of the covering. The cross-linking can be covalent, ionic, or both. For reference regarding the application of layers of heparin to the membrane 260, reference may be made to U.S. Patent No. 9,399,085 (Cleek et al*.*), which is incorporated herein by reference. The device implants with the CBAS^{®} Heparin Surface did have evidence of non-specific entrapment of red blood cells in the film's microstructure (as discussed in further detail below with reference to FIG. 16 A and FIG. 16B).

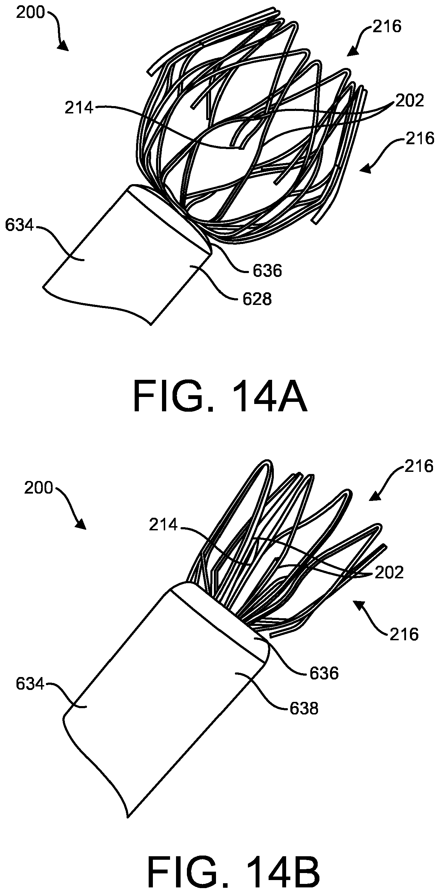

FIG. 14A shows an example frame 200 and anchors 214 with a sheath 634 (or delivery device such as a delivery catheter) in a first configuration, in accordance with various aspects of the present disclosure. The first configuration shown in FIG. 14A may be considered a deployed configuration of the frame 200. The anchors 214 project outwardly relative to the frame 200. The deployed configuration may be prior to the frame 200 being loaded into the sheath 634 for implantation into the body, and also after the frame 200 is unloaded or deployed from the sheath 634 into the body.

FIG. 14B shows the frame 200, anchors 214, and the sheath 634, as shown in FIG. 14A, in a second configuration, in accordance with various aspects of the present disclosure. The second configuration shows the frame 200 being withdrawn into or deployed from the sheath 634. The frame 200 is not completely in a delivery configuration (*e.g*., within the sheath 634). As shown in comparing FIG. 14A and 14B, the anchors 214, which had been projecting outwardly the deployed configuration shown in FIG. 14A, have moved inwardly (*e.g*., rotated) relative to and toward the longitudinal axis in response to the frame 200 being withdrawn into the sheath 634. The anchors 214, in certain embodiments, are substantially aligned with the adjacent struts 202 in the delivery configuration.

The sheath 634 includes a substantially circular body portion 636 at a distal end of the sheath 634. The substantially circular body portion 636 is an entry/exit point for a lumen 638 into which the frame 200 may be withdrawn for subsequent deployment or redeployment of the frame 200. As shown in FIG. 14B, the anchors 214, in response to the frame 200 being transitioned to the delivery configuration, the anchors 214 are configured to rotate inwardly such that the anchors 214 do not contact the substantially circular body portion 636 (or other portions of) the sheath 634. Portions of the anchors 214 (such as the anchor tips) also may avoid contact with the lumen 638 of the sheath 634 when withdrawn therein. In the absence of the anchors 214 being configured to rotate inwardly in this manner, a user operating the sheath 634 would encounter a large resistance when attempting to withdraw the frame 200 in the sheath 634. The anchors 214 being configured to rotate inwardly avoids unnecessary resistance in the deployment process. The unnecessary resistance could also damage the frame 200 itself by irreparably pleating or folding the frame 200. Thus, the anchors 214 being configured to rotate inwardly in this manner avoids damaging the sheath 634, avoids damaging the frame 200, and eases delivery and deployment of the implantable medical devices that include the frame 200. In certain instances, tips of the anchors 214 avoid contact with the sheath 634. The anchors 214 rotating when transitioned to the delivery configuration brings a tip (which may be approximately 1%, 2%, 3%, 4%, 5%, 6%, 7%, 8%, 9%, 10% of a length of the anchor 206 as measured from the distal end) away from an out of contact with the sheath 634. In other instances, the anchors 214 rotating in this manner brings the tip to the interior of the frame 200.

The anchors 214 are also configured to move outwardly from the longitudinal axis in response to deploying the frame 200 from the sheath 634. In addition to demonstrating the configuration or positioning changes of the anchors 214 when the frame transitions from the deployed configuration to the delivery configuration, the outward deflection of the anchors 214 is demonstrated in comparing the second configuration in FIG. 14B to the first configuration in FIG.14A. As shown in FIG. 14B, the distal end 416 of the frame has expanded as it is retracted into the sheath 634. The open distal end 416 facilitates transmission of the collapsing force throughout the entirety of the frame to allow the frame 200 to collapse into an elongated delivery configuration. In this manner, the frame 200 collapses by retracting into the sheath 634 without the need for the application of a collapsing force to the distal end 416. The open distal end 416 lessens elongation during deployment and while the device 30 is arranged within a patient. In certain instances, the open distal end 416 may be between about 4 mm to 8 mm in diameter.

The sheath 634 is configured to deploy a distal end 416 of the frame prior 200 to the proximal end (shown contacting the substantially circular body portion 636 of the sheath 634 in FIG. 14A) of the frame 200. In addition, the anchors 214 project outwardly (from a waist portion 210) and curve upward toward the proximal end in the deployed configuration shown in FIG. 14A. The anchors 214 move outwardly in response to deploying the frame 200 from the sheath 634.

In certain embodiments, a user of the sheath 634 may recapture the frame 200 (and implantable medical device) within the sheath 634. After implanting the frame 200 within the body by deploying the frame 200 from the sheath 634 and expanding the frame 200 to the deployed configuration, placement of the frame 200 may not be in the intended location or at the intended angle. Thus, the user may wish to recapture and redeploy the frame 200. In these embodiments, the sheath 634 is configured to withdraw the frame 200 into the sheath 634 (into the delivery configuration) with the anchors 214 being configured to rotate radially inwardly and disengage from the tissue in the body. The anchors 214 atraumatically disengage from the tissue due to the retracting motion.

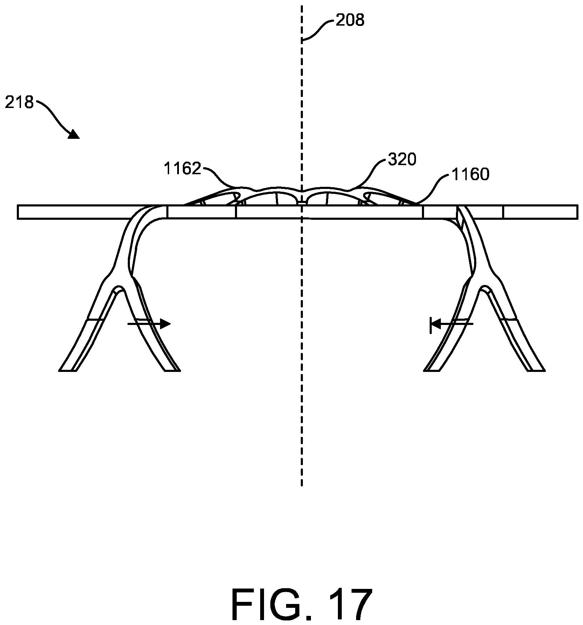

FIG. 17 shows another example frame 200 for an implantable medical device, in accordance with various aspects of the present disclosure. In certain instances, the frame 200 may include a raised portion 1160 that is an increase in height relative to a longitudinal axis 208 and other portions of the proximal end 218 of the frame 200.

In certain instances, the raised portion 1160 includes a diameter that is approximately about 5%, about 10%, about 15% or any number therebetween of a diameter of the proximal end 218 of the frame 200. In addition, the raised portion 1160 may include a peak in height at the center frame portion 320. The raised portion 1160 may include a gradual increase in height relative to other portions of the proximal end 218 of the frame 200. In certain instances, the raised portion 1160 includes between a 15 degree to a 25 degree angle 1162 relative to the other portions of the proximal end 218 of the frame 200. In some embodiments, the raised portion 1160 has about a 16 degree, about 17 degree, about 18 degree, about 19 degree, about 20 degree, about 21 degree, about 22 degree, about 23 degree, about 24 degree or about 25 degree increase relative to the other portions of the proximal end 218 of the frame 200. The angle 1162 being gradual facilitates the proximal end 218 maintaining a uniform surface by not creating a protrusion or abrupt increase in height in the proximal end 218 of the frame 200. In some instances, the center frame portion 320 has a thickness equal to a thickness of at least a majority of the implantable device. In addition, the increase in height relative to other portions of the proximal end 218 of the frame 200 of the raised portion 1160 may be between about 0.1mm and about 0.6 mm. For example, the height relative to other portions may be about 0.25mm.

In certain instances, the raised portion 1160 may facilitate transitioning of the frame 200 into a collapsed configuration. The raised portion 1160 lessen strain on the center frame portion 320 in transitioning to the collapsed configuration within a delivery sheath by distributing strain about the center frame portion 320 and/or the proximal end 218.

Suitable membrane materials for the membrane 260 include, but are not limited to, polymers such as olefin, PEEK, polyamide, polyurethane, polyester, such as polyethylene terephthalate (PET), polyethylene, polypropylene, polyurethane, silicone, fluorinated ethylene propylene (FEP), polytetrafluoroethylene (PTFE), expanded polytetrafluoroethylene (ePTFE), and fluoroelastomers, such as tetrafluoroethylene/polymethylvinylether (TFE/PMVE) copolymers. In certain instances, the second layer 414 of the membrane 260 is the elastomeric material (e.g., TFE/PMVE). The second layer 414 may be configured to reinforce the membrane 260 and facilitate deflection and rebounding of the membrane 260.

In certain instances, the second layer 414 may be attached, adhered or coupled to the first layer 340, to at least a portion of each of the face portions of the elongate members 212 or both the first layer 340 and at least a portion of each of the face portions of the elongate members 212. The second layer 414 may be attached, adhered or coupled to the first layer 340 only at the perimeter of the second layer 414. This may further facilitate the rebounding effect of the membrane 260.

A biocompatible material for the graft components, discussed herein, may be used. In certain instances, the graft may include a fluoropolymer, such as a polytetrafluoroethylene (PTFE) polymer or an expanded polytetrafluoroethylene (ePTFE) polymer. In some instances, the graft may be formed of, such as, but not limited to, a polyester, a silicone, a urethane, a polyethylene terephthalate, or another biocompatible polymer, or combinations thereof. In some instances, bioresorbable or bioabsorbable materials may be used, for example a bioresorbable or bioabsorbable polymer. In some instances, the graft can include Dacron, polyolefins, carboxy methylcellulose fabrics, polyurethanes, or other woven, non-woven, or film elastomers.

In addition, nitinol (NiTi) may be used as the material of the frame or stent (and any of the frames discussed herein), but other materials such as, but not limited to, stainless steel, L605 steel, polymers, MP35N steel, polymeric materials, Pyhnox, Elgiloy, or any other appropriate biocompatible material, and combinations thereof, can be used as the material of the frame. The super-elastic properties and softness of NiTi may enhance the conformability of the stent. In addition, NiTi can be shape-set into a desired shape. That is, NiTi can be shape-set so that the frame tends to self-expand into a desired shape when the frame is unconstrained, such as when the frame is deployed out from a delivery system.

Several implantable occlusive device and frame embodiments have been described herein. It should be understood that one or more of the features described in the context of a particular device may be combined with one or more features of any other device or multiple devices described herein. That is, the features of the occlusive devices and frames described herein may be mixed and matched to provide hybrid occlusive device and device frame embodiments, and such hybrid occlusive device and device frame embodiments are within the scope of this disclosure. In some examples, one or more features described with respect to a particular device or frame may replace or be substituted for one or more features of another device or frame. In some examples, one or more features described with respect to a particular device or frame may be added to or included with another device or frame. Also, various combinations or sub-combinations of any of the features described herein may generally be used with any of the devices or frames described herein. It should be understood that the occlusive devices and occlusive device frames provided herein are scalable to a broad range of sizes so that the occlusive devices can be used in a variety of different anatomies, implant sites, and types of implementations.

In certain instances, the coating, as discussed in detail above, may include bio-active agents in addition to heparin or alternatively to heparin. The agents can include, but are not limited to, vasodilator, anti-coagulants, anti-platelet, anti-thrombogenic agents.

### TEST METHODS

It should be understood that although certain methods and equipment are described below, other methods or equipment determined suitable by one of ordinary skill in the art may be alternatively utilized.

### Test Methods

Evaluations of heparin coated devices were performed in an acute in vitro re-circulating blood contact model called the Chandler Loop or "blood loop". This acute blood contact model has an established history as a sensitive method for assessing thrombogenicity (Chandler AB, 1958, Sanchez et al, 1997, Andersson et al, 2003, Sinn et al. 2011). The method simulates the circulation of blood by rotating closed tubing loops. In vitro blood contact testing allows for determination of relative thrombogenicity of implant surfaces. The Chandler loop model utilizes direct implant contact with circulating blood. When fresh, non-anticoagulated, non-heparinized human blood is used, this model can discriminate thromboresistant from non-thrombogenic surfaces.

As one example of tested devices, control and test implants were be deployed into HeartPrint^{®} Flex Connectors having the CBAS^{®} Heparin Surface. The loops were completed with PVC tubing (inner diameter of 8mm) having the CBAS^{®} Heparin Surface which eliminated the HeartPrint^{®} Flex and PVC surface as a trigger for any observed initiation of the clotting cascade or platelet activation. In addition, one 3mm PVC tubing segment with the CBAS^{®} Heparin Surface, one 3mm dextran sulfate-coated (DS) PVC tubing segment, and one 3mm uncoated PVC tubing segment, all without an implant, served as system controls in each experiment. The implants and system controls were then be exposed to non-anticoagulated human whole blood in a blood loop for two hours.

The PVC tubing segments coated with CBAS^{®} Heparin Surface), with and without deployed implants, were wet out with IPA. The loops were then rinsed with water and then 0.15M NaCl (physiological saline) solution overnight to remove any residual unbound CBAS^{®}-heparin which may be present on the surface of the implant and the tubing. Also, the tubing segments of dextran sulfate-coated and uncoated PVC (Loops 5 and 6) were rinsed overnight with 0.15M NaCl prior to the blood contact experiments.

Following the two hour blood loop evaluation, loops were drained of the whole blood and gently flushed with saline to remove any non-adhered blood components. The drained blood (and any blood which has seeped behind the implants) was collected in a tube containing EDTA anticoagulant in order to measure platelet counts in whole blood and biomarkers in plasma. The implants were removed from the HeartPrint^{®} Flex connectors, further rinsed, photographed, and fixed in formalin. The fixed implants and plasma aliquots were used for SEM evaluation and measurement of biomarkers, respectively.

FIG. 16A is a representative SEM image (1000X) of the face of a device uncoated implant. FIG. 16B is a representative SEM image (1000X) of the face of a device implant with the CBAS^{®} Heparin Surface. In general, the device with the CBAS^{®} Heparin Surface had less evidence of thrombus elements such as fibrin and activated platelets. The device implants with the CBAS^{®} Heparin Surface did have evidence of non-specific entrapment of red blood cells in the film's microstructure (FIG. 16B and FIG. 13B). In contrast, the uncoated device implant faces showed evidence of thrombus elements (accumulation of fibrin and entrapped blood cells, including both platelets and red blood cells), both isolated and extensive as shown in FIG. 16A and FIG. 13C.

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims and their equivalents.

### Clauses

1. A device for placement in vessels, appendages, and openings in a body, the device comprising:
   a frame having a plurality of elongate members, the frame including:
      a substantially uniform proximal end of the frame having (i) a center frame portion arranged at the proximal end and (ii) face portions of the plurality of elongate members extending from the center frame portion and including a curved pattern arranged substantially within a first plane approximately perpendicular to a longitudinal axis of the frame;
      a body portion;
   a membrane coupled to at least the substantially uniform proximal end of the frame; and
   a coating arranged on at least a portion of the membrane configured to minimize the thrombogenic response of blood contact with the membrane.
2. The device of clause 1, wherein the coating comprises heparin configured to lessen blood coagulation along the membrane coupled to at least the substantially uniform proximal end of the frame.
3. The device of clause 1, wherein the coating is configured to decrease platelet adhesion to an exterior surface of the membrane coupled to at least the substantially uniform proximal end of the frame.
4. The device of clause 1, wherein the coating is a carmeda heparin coating.
5. The device of clause 1, wherein the plurality of elongate members are configured to angularly displace the center frame portion by between about 30 degrees and about 45 degrees in response to a compressive force applied to the body portion of the frame.
6. The device of clause 1, wherein the plurality of elongate members are configured to angularly displace the center frame portion by between about 30 degrees and about 45 degrees in response to the compressive force reducing the circumference of the body portion of the frame by between about 20% and about 50%.
7. The device of clause 6, wherein the plurality of elongate members are configured to angularly displace the center frame portion and maintain the center frame portion approximately aligned with a longitudinal axis of the frame in response to the compressive force.
8. The device of clause 1, wherein the face portions of the plurality of elongate members include a first curved section, a second curved section, and an inflection point between the first curved section and the second curved section within the proximal end of the frame.
9. The device of clause 1, wherein the body portion of the frame includes a shoulder portion including first curved portions of the plurality of elongate members transitioning from the first plane to align approximately parallel with the longitudinal axis of the frame, and a first body portion including a first plurality of cells arranged circumferentially about the frame formed by first body portions of the plurality of elongate members, a hip portion including second curved portions of the plurality of elongate members transitioning the frame inwardly toward the longitudinal axis of the frame, and a second body portion including a second plurality of cells arranged circumferentially about the frame.
10. The device of clause 9, wherein the membrane includes a plurality of openings arranged circumferentially about the frame.
11. The device of clause 10, wherein the frame includes a plurality of anchors arranged at the hip portion configured to rotate relative to and toward the longitudinal axis and pass through the plurality of openings.
12. The device of clause 11, wherein the plurality of anchors are configured to rotate and pass through the plurality of openings in response to constriction of the substantially uniform proximal end of the frame.
13. The device of clause 12, wherein the plurality of anchors are configured to rotate pass through the plurality of openings in in response to the frame being arranged in a delivery configuration to avoid contact between an anchor tip and a delivery sheath.
14. A method of reducing thrombus arising from occluding a left atrial appendage of a patient, the method comprising:
   placing the implantable medical device across an ostium of the left atrial appendage, the implantable medical device having a frame with a substantially uniform proximal end and a membrane coupled to at least the substantially uniform proximal end of the frame and a coating arranged on at least a portion of the membrane configured to minimize the thrombogenic response of blood contact with the membrane.
15. The method of clause 14, wherein the coating comprises heparin configured to lessen blood coagulation along the membrane coupled to at least the substantially uniform proximal end of the frame and decrease platelet adhesion to an exterior surface of the membrane coupled to at least the substantially uniform proximal end of the frame.

## Claims

1. A device for placement in vessels, appendages, and openings in a body comprising:
a frame having a plurality of elongate members, the frame including:
a substantially uniform proximal end of the frame having
(i) a center frame portion arranged at the proximal end and
(ii) face portions of the plurality of elongate members extending from the center frame portion and including a curved pattern arranged substantially within a first plane approximately perpendicular to a longitudinal axis of the frame, a body portion including first body portions of the plurality of elongate members arranged substantially parallel to the longitudinal axis of the frame and second body portions of the plurality of elongate members tapering the frame inwardly relative to the longitudinal axis; and a membrane attached to at least a portion of the frame and including a plurality of openings arranged circumferentially about the frame; and a plurality of anchors arranged at the hip portion configured to rotate relative to and toward the longitudinal axis and pass through the plurality of openings.

2. The device of claim 1, wherein the plurality of anchors are configured to rotate and pass through the plurality of openings in response to constriction of the substantially uniform proximal end of the frame.

3. The device of claim 1, the plurality of anchors are configured to rotate pass through the plurality of openings in in response to the frame being arranged in a delivery configuration to avoid contact between an anchor tip and a delivery sheath.

4. A device for placement in vessels, appendages, and openings in a body including a frame having a plurality of elongate members, the frame including:
a substantially uniform proximal end of the frame having
(i) a center frame portion arranged at the proximal end and
(ii) face portions of the plurality of elongate members extending from the center frame portion; a body portion, and a membrane attached to the frame and the plurality of elongate members are configured to angularly displace the center frame portion by between about 30 degrees and about 45 degrees in response to a compressive force applied to the body portion of the frame.

5. The device of claim 4, wherein the plurality of elongate members are configured to angularly displace the center frame portion by between about 30 degrees and about 45 degrees in response to the compressive force reducing the circumference of the body portion of the frame by between about 20% and about 50%.

6. The device of claim 5, wherein the plurality of elongate members are configured to angularly displace the center frame portion and maintain the center frame portion approximately aligned with a longitudinal axis of the frame in response to the compressive force.

7. The device of claim 4, wherein the face portions of the plurality of elongate members include a first curved section, a second curved section, and an inflection point between the first curved section and the second curved section within the proximal end of the frame.

8. The device of claim 7, wherein the face portions of the plurality of elongate members each include a starting point at the center frame portion and an end point a perimeter of the proximal end and the starting point and the ending port are radially offset relative to a tangent between the starting point and the ending point.

9. The device of claim 8, wherein the starting point of the face portions of the elongate members and the end point of the face portions of the elongate members are offset by about 40 degrees and about 60 degrees.

10. The device of claim 8, wherein a tangent perpendicular to the starting point at the center frame portion of each of the face portions of the elongate members intersects the perimeter of the proximal end between two of the face portions of the plurality of elongate members.

11. The device of claim 7, wherein the first curved section includes a first radius of curvature and the second curved section includes a second radius of curvature and the first radius of curvature and the second radius of curvature are approximately equal.

12. The device of claim 7, wherein the first curved section and the second curved section are in opposite directions.

13. The device of claim 4, wherein the body portion includes a distal section including one or more circumferential members.

14. The device of claim 4, wherein the circumferential members in the distal section facilitate uniform compression of the frame.
